# EUROPEAN PATENT SPECIFICATION

(11) **EP 1 814 852 B1**
(45) Date of publication and mention of the grant of the patent: **20.01.2016**
(21) Application number: 05819967.0
(22) Date of filing: 09.11.2005
(51) Int. Cl.: C07D 213/46, A61K 31/4406

(54) **HYDROXYBENZOATE SALTS OF METANICOTINE COMPOUNDS**
HYDROXYBENZOATSALZE VON METANICOTINVERBINDUNGEN
SELS D'HYDROXYBENZOATE DE COMPOSES DE METANICOTINE

(30) Priority: 10.11.2004 US 626751 P
(43) Date of publication of application: 08.08.2007
(60) Divisional application: 11002581.4
(73) Proprietor: Catalyst Biosciences, Inc., South San Francisco, CA 94080 (US)
(72) Inventor: MUNOZ, Julio, A., Walnut Cove, NC 27052 (US); GENUS, John, Winston-Salem NC 27127 (US); MOORE, James, R., Newark, DE 19702 (US)
(74) Representative: Potter Clarkson LLP
(86) International application number: PCT/US2005/040650
(87) International publication number: WO 2006/053082

(56) References cited:
- US-B1- 6 232 316
- US-B1- 6 432 954
- US-B1- 6 492 399

## Description

### Field of the Invention

The present invention relates to processes for preparing nicotinic compounds and pharmaceutically acceptable salts thereof, as well as pharmaceutical compositions and methods for treating a wide variety of conditions and disorders associated with dysfunction of the central and autonomic nervous systems.

### Background of the Invention

Nicotine has been proposed to have a number of pharmacological effects. See, for example, Pullan et al., N. Engl. J. Med. 330:811-815 (1994). Certain of those effects can be related to effects upon neurotransmitter release. Release of acetylcholine, dopamine, norepinephrine, serotonin, and glutamate upon administration of nicotine has been reported (Rowell et al., J. Neurochem. 43:1593 (1984); Rapier et al., J. Neurochem. 50:1123 (1988); Sandor et al., Brain Res. 567:313 (1991); Vizi, Br. J. Pharmacol. 47:765 (1973); Hall et al., Biochem. Pharmacol. 21:1829 (1972); Hery et al., Arch. Int. Pharmacodyn. Ther. 296:91 (1977); and Toth et al., Neurochem Res. 17:265 (1992)). Confirmatory reports and additional recent studies have included the modulation in the Central Nervous System (CNS) of glutamate, nitric oxide, GABA, takykinins, cytokines, and peptides (reviewed in Brioni et al., Adv. Pharmacol. 37:153 (1997)). In addition, nicotine reportedly potentiates the pharmacological behavior of certain pharmaceutical compositions used to treat certain disorders. See, for example, Sanberg et al., Pharmacol. Biochem. & Behavior 46:303 (1993); Harsing et al., J. Neurochem. 59:48 (1993); and Hughes, Proceedings from Intl. Symp. Nic. S40 (1994). Furthermore, the neuroprotective effects of nicotine have been proposed, see, for example, Sjak-shie et al., Brain Res. 624:295 (1993). Various other beneficial pharmacological effects have also been proposed. See, for example, Decina et al., Biol. Psychiatry 28:502 (1990); Wagner et al., Pharmacopsychiatry 21:301 (1988); Pomerleau et al., Addictive Behaviors 9:265 (1984); Onaivi et al., Life Sci. 54(3):193 (1994); Tripathi et al., J. Pharmacol. Exp. Ther. 221:91 (1982); and Hamon, Trends in Pharmacol. Res. 15:36 (1994).

Various compounds that target nAChRs (nicotinic acetylcholinergic receptors) have been reported as being useful for treating a wide variety of conditions and disorders. See, for example, Williams et al., DN&P 7(4):205 (1994); Arneric et a/., CNS Drug Rev. 1(1):1 (1995); Arneric et al., Exp. Opin. Invest. Drugs 5(1):79 (1996); Bencherif et al., J. Pharmacol. Exp. Ther. 279:1-13 (1996); Lippiello et al., J. Pharmacol. Exp. Ther. 279:1422 (1996); Damaj et al., J. Pharmacol. Exp. Ther. 291:390 (1999); Chiari et al., Anesthesiology 91:1447 (1999); Lavand'homme and Eisenbach, Anesthesiology 91:1455 (1999); Holladay et al., J. Med. Chem. 40(28): 4169 (1997); Bannon et al., Science 279:77 (1998); PCT WO 94/08992; PCT WO 96/31475; PCT WO 96/40682; and U.S. Patent Nos. 5,583,140 to Bencherif et al.; 5,597,919 to Dull et al.; 5,604,231 to Smith et al.; and 5,852,041 to Cosford et al. US 6,432,954 describes the treatment of central nervous system disorders by the administration of 2,3-diacyltartaric acid salts of nicotinic compounds. US 6,232,316 and US 6,492,399 describe pharmaceutical compositions incorporating aryl substituted olefinic amine compounds. Nicotinic compounds are reported as being particularly useful for treating a wide variety of CNS disorders. Indeed, a wide variety of nicotinic compounds have been reported to have therapeutic properties. See, for example, Bencherif and Schmitt, Current Drug Targets: CNS and Neurological Disorders 1(4): 349-357 (2002), Levin and Rezvani, Current Drug Targets: CNS and Neurological Disorders 1(4): 423-431 (2002), O'Neill, et al., Current Drug Targets: CNS and Neurological Disorders 1(4): 399-411 (2002), U.S. Patent Nos. 5,187,166 to Kikuchi et al.*,* 5,672,601 to Cignarella, PCT WO 99/21834 and PCT WO 97/40049, UK Patent Application GB 2295387 and European Patent Application 297,858.

CNS disorders are a type of neurological disorder. CNS disorders can be drug-induced; can be attributed to genetic predisposition, infection or trauma; or can be of unknown etiology. CNS disorders comprise neuropsychiatric disorders, neurological diseases, and mental illnesses, and include neurodegenerative diseases, behavioural disorders, cognitive disorders, and cognitive affective disorders. There are several CNS disorders whose clinical manifestations have been attributed to CNS dysfunction (*i.e*., disorders resulting from inappropriate levels of neurotransmitter release, inappropriate properties of neurotransmitter receptors, and/or inappropriate interaction between neurotransmitters and neurotransmitter receptors). Several CNS disorders can be attributed to a deficiency of acetylcholine, dopamine, norepinephrine, and/or serotonin.

Relatively common CNS disorders include pre-senile dementia (early-onset Alzheimer's disease), senile dementia (dementia of the Alzheimer's type), micro-infarct dementia, AIDS-related dementia, vascular dementia, Creutzfeld-Jakob disease, Pick's disease, Parkinsonism including Parkinson's disease, Lewy body dementia, progressive supranuclear palsy, Huntington's chorea, tardive dyskinesia, hyperkinesia, epilepsy, mania, attention deficit disorder, anxiety, dyslexia, schizophrenia, depression, obsessive-compulsive disorders, and Tourette's syndrome.

Subtypes of nAChRs are present in both the central and peripheral nervous systems, but the distribution of subtypes is heterogeneous. For instance, the subtypes which are predominant in vertebrate brain are α4β2, α7, and α3β2, whereas those which predominate at the autonomic ganglia are α3β4 and those of neuromuscular junction are α1β1δγ and α1β1δε (see for instance Dwoskin et al., Exp. Opin. Ther. Patents 10: 1561 (2000); and Schmitt and Bencherif, Annual Reports in Med. Chem. 35: 41 (2000)).

A limitation of some nicotinic compounds is that they elicit various undesirable pharmacological effects because of their interaction with nAChRs in peripheral tissues (for example, by stimulating muscle and ganglionic nAChR subtypes). It is therefore desirable to have compounds, compositions, and methods for preventing and/or treating various conditions or disorders (*e.g*., CNS disorders), including alleviating the symptoms of these disorders, where the compounds exhibit nicotinic pharmacology with a beneficial effect on the CNS nAChRs (*e.g*., upon the functioning of the CNS), but without significant associated effects on the peripheral nAChRs (compounds specific for CNS nAChRs). It is also highly desirable to have compounds, compositions, and methods that affect CNS function without significantly affecting those receptor subtypes which have the potential to induce undesirable side effects (*e.g*., appreciable activity at cardiovascular and skeletal muscle sites).

Methods for treating and/or preventing the above-described conditions and disorders by administering E-metanicotine compounds, particularly those which maximize the effect on CNS function without significantly affecting those receptor subtypes which have the potential to induce undesirable side effects, have been described in the art. Representative E-metanicotine compounds for use in treating and/or preventing the above-described disorders are disclosed, for example, in U.S. Pat. No. 5,212,188 to Caldwell et al.*,* U.S. Pat. No. 5,604,231 to Smith et al.*,* U.S. Pat. No. 5,616,707 to Crooks et al.; U.S. Pat No. 5,616,716 to Dull et al, No. 5,663,356 to Ruecroft et al*,* US Pat. No. 5,726,316 to Crooks et al*,* US Pat. No. 5,811,442 to Bencherif et al*,* US Pat. No. 5,861,423 to Caldwell et al*,* PCT WO 97/40011; PCT WO 99/65876 PCT WO 00/007600; and US patent application Ser. No. 09/391,747, filed on Sep. 8, 1999.

The syntheses described in the art for forming E-metanicotines typically involve performing a Heck reaction between a halogenated heteroaryl ring, such as a halo-pyridine or halo-pyrimidine, and a double bond-containing compound. The double bond-containing compound typically includes either a hydroxy group, which is converted to an amine group to form the E-metanicotine, or includes a protected amine group, which is deprotected following the Heck reaction to form the E-metanicotine. A limitation of the Heck coupling chemistry is that, while the major reaction product is the desired E-metanicotine, there are minor reaction products, including the Z-metanicotine, a metanicotine compound where the double bond has migrated from the position adjacent to the heteroaryl (such as pyridine or pyrimidine) ring (i.e., a non-conjugated double bond), and a compound in which the heteroaryl group is attached at the secondary (as opposed to primary) alkene carbon (i.e., a methylene compound or "exo" double bond). It can be difficult to remove these minor reaction products, particularly on scale-up.

It would be advantageous to provide new methods of preparing purified E-metanicotine compounds substantially free from the above-described minor reaction products. It would also be advantageous to provide new salt forms of these drugs to improve their bioavailability, and/or to assist in preparing large quantities of these compounds in a commercially reasonable manner. The present invention provides such new synthesis methods and new salt forms.

### Summary of the Invention

The invention provides a salt formed as the reaction product of (4E)-N-methyl-5-(5-isoproxy-3-pyridinyl)-4-penten-2-amine and a hydroxybenzoic salt as defined in claim 1, and a process for preparing (2S)-(4E)-N-methyl-5-(5-isopropoxy-3-pyridinyl-4-pentene-2-amine or a corresponding hydroxybenzoate salt as defined in claim 7.

New methods of synthesizing E-metanicotine compounds are also described herein, as well as new pharmaceutically acceptable salt forms of E-metanicotine compounds. Pharmaceutical compositions including the new salt forms, and methods of treatment and/or prevention using the new salt forms, are also disclosed. However, it is to be understood that the scope of the invention does not cover all E-metanicotine compounds as described herein, but is limited to the (4E)-N-methyl-5-(5-isopropoxy-3-pyridinyl)-4-pentene-2-amine compounds defined in claims 1 and 7.

The methods for synthesizing the E-metanicotine compounds typically include the step of performing a Heck reaction between a halogenated heteroaryl ring, such as a halo-pyridine or halo-pyrimidine, and a double bond-containing compound. The double bond-containing compound typically includes either a hydroxy group, which is subsequently converted to an amine group to form the E-metanicotine compound, or includes a protected amine group, which is deprotected following the Heck reaction to form the E-metanicotine compound.

After the Heck reaction and formation of an E-metanicotine with a free amine group (whether by conversion of a hydroxy group or deprotection of a protected amine group), the next step involves forming a hydroxybenzoate salt of the E-metanicotine compound. Under certain conditions, one can precipitate out the hydroxybenzoate salt of the E-metanicotine compound while leaving the minor impurities (Z-metanicotine and/or the isomers of the E-metanicotine compound wherein the double bond has migrated to a position other than directly adjacent to the heteroaryl ring or wherein the attachment of the aryl group to the alkene chain is at the secondary double bond carbon) in solution. This improvement makes it relatively easy to remove these minor reaction products, particularly on scale-up.

The synthesis of the E-metanicotines typically involves forming an amine-protected 4-penten-2-amine intermediate, and coupling this intermediate via a Heck reaction with a halogenated heteroaryl ring. The choice of heteroaryl ring is not essential to the success of the Heck coupling reaction, although pyridine and pyrimidine rings can be preferred. (2S)-(4E)-N-methyl-5-[3-(5-isopropoxypyridin)yl)]-4-penten-2-amine is a representative E-metanicotine, p-hydroxybenzoate is a representative hydroxybenzoate salt, and (2S)-(4B)-N-methyl-5-[3-(5-isopropoxypyridin)yl)]-4-penten-2-amine p-hydroxybenzoate is a representative E-metanicotine hydroxybenzoate salt.

An exemplary reaction is shown below:
Cy-Hal + CH₂=CH-CH₂CH(CH₃)N(CH₃)(tBoc) →
(E) Cy-CH=CH-CH₂CH(CH₃)N(CH₃)(tBoc)
+ (Z) Cy-CH=CH-CH₂CH(CH₃)N(CH₃)(tBoc)
+ (E and/or Z) Cy-CH₂CH=CHCH(CH₃)N(CH₃)(tBoc)
+ Cy-C(=CH₂)-CH₂CH(CH₃)N(CH₃)(tBoc)
where Cy is a five or six membered heteroaryl ring.

Alternatively, the Heck coupling reaction takes place using a hydroxy-alkene, such as 4-penten-2-ol, and the hydroxy group is converted to an amine group after the Heck coupling reaction takes place. The conversion can be effected, for example, by converting the hydroxy group to a tosylate, and displacing the tosylate with a suitable amine, such as methylamine. The Heck coupling reaction still forms the same major and minor products, except that they include a hydroxy group rather than a protected amine group. Following formation of the amine-containing compound (i.e., the (E)-metanicotine), if the impurities (i.e., the minor products of the Heck coupling reaction) are not already removed, the chemistry involved in forming the hydroxybenzoate salts is substantially the same.

After deprotecting the amine group, or forming the amine group, one can form a hydroxybenzoate salt of the E-metanicotine by reaction with a hydroxybenzoic acid as described herein. The hydroxybenzoate salts of the major product (the (E)-metanicotine) and of the minor products will form. However, under certain conditions, the hydroxybenzoate salt of the major reaction product, the (E)-metanicotine hydroxybenzoate salt, will precipitate out of solution in relatively pure form, leaving behind a mother liquor enriched in the minor impurities. This result comprises a significant advance in the synthesis and purification of (E)-metanicotines.

The hydroxybenzoate salts typically are isolated and then used as intermediates to form different salt forms by reaction with different pharmaceutically acceptable acids or salts thereof. However, alternatively, the E-metanicotine hydroxybenzoate salts are used as active pharmaceutical ingredients (API's). The hydroxybenzoate salts can be used directly, or included in pharmaceutical compositions by combining them with a pharmaceutically acceptable excipient. The hydroxybenzoate salts and/or pharmaceutical compositions can be used to treat and/or prevent a wide variety of conditions or disorders. The disorders are particularly those disorders characterized by dysfunction of nicotinic cholinergic neurotransmission, including disorders involving neuromodulation of neurotransmitter release, such as dopamine release. The compounds can be used in methods for treatment and/or prophylaxis of disorders, such as central nervous system (CNS) disorders, which are characterized by an alteration in normal neurotransmitter release. The compounds can also be used to treat certain conditions (e.g., a method for alleviating pain). The methods involve administering to a subject an effective amount of a E-metanicotine hydroxybenzoate salt, or pharmaceutical composition including a E-metanicotine hydroxybenzoate salt, as described herein.

The pharmaceutical compositions, when employed in effective amounts, can interact with relevant nicotinic receptor sites in a patient, and act as therapeutic and/or prophylactic agents in connection with a wide variety of conditions and disorders, particularly CNS disorders characterized by an alteration in normal neurotransmitter release. The pharmaceutical compositions can provide therapeutic benefit to individuals suffering from such disorders and exhibiting clinical manifestations of such disorders in that the compounds within those compositions, when employed in effective amounts, can (i) exhibit nicotinic pharmacology and affect relevant nicotinic receptors sites (e.g., activate nicotinic receptors), and (ii) modulate neurotransmitter secretion, and hence prevent and suppress the symptoms associated with those disorders. In addition, the compounds can (i) increase the number of nicotinic cholinergic receptors of the brain of the patient, (ii) exhibit neuroprotective effects and (iii) when employed in effective amounts can exhibit relatively low levels of adverse side effects (e.g., significant increases in blood pressure and heart rate, significant negative effects upon the gastro-intestinal tract, and significant effects upon skeletal muscle).

The foregoing and other aspects of the present invention are explained in detail in the detailed description and examples set forth below.

### Detailed Description of the Invention

The hydroxybenzoate salts described herein, which are derived from E-metanicotines and hydroxybenzoic acids, have a number of advantages over other salts derived from E-metanicotines and other acids. In general, the hydroxybenzoic acid salts of E-metanicotines are water-soluble materials that tend to be highly crystalline and less hygroscopic in nature than other salts. For example, the p-hydroxybenzoate salt of (2S)-(4E)-N-methyl-5-[3-(5-isopropoxypyridin)yl)]-4-penten-2-amine is physically and chemically stable, free-flowing, crystalline powder. Such properties are definite advantages for pharmaceutical formulation development and pharmaceutical manufacturing. If necessary, this salt can be milled to an acceptable particle size range for pharmaceutical processing. The salt is compatible with a wide range of excipients that might be chosen for the manufacture of solid oral dosage forms. This is especially so for those exicipients, such as polysaccharide derivatives, that are pharmaceutically defined hydrates and those with only loosely bound surface water. As an illustration, salts derived from certain E-metanicotines, such as E-metanicotine and fumaric acid are prone to the formation of impurities within the salt. For example, impurities arise from the Michael addition reaction of the secondary amine in E-metanicotine to the olefin in fumaric acid. These impurities lower the chemical purity of the salt and adversely affect the chemical integrity of the salt upon long-term strorage.

As used herein, an "agonist" is a substance that stimulates its binding partner, typically a receptor. Stimulation is defined in the context of the particuar assay, or may be apparent in the literature from a discussion herein that makes a comparison to a factor or substance that is accepted as an "agonist" or an "antagonist" of the particular binding partner under substantially similar circumstances as appreciated by those of skill in the art. Stimulation may be defined with respect to an increase in a particular effect or function that is induced by interaction of the agonist or partial agonist with a binding partner and can include allosteric effects.

As used herein, an "antagonist" is a substance that inhibits its binding partner, typically a receptor. Inhibition is defined in the context of the particular assay, or may be apparent in the literature from a discussion herein that makes a comparison to a factor or substance that is accepted as an "agonist" or an "antagonist" of the particular binding partner under substantially similar circumstances as appreciated by those of skill in the art.

Inhibition may be defined with respect to a decrease in a particular effect or function that is induced by interaction of the antagonist with a binding partner, and can include allosteric effects.

As used herein, a "partial agonist" is a substance that provides a level of stimulation to its binding partner that is intermediate between that of a full or complete antagonist and an agonist defined by any accepted standard for agonist activity. It will be recognized that stimulation, and hence, inhibition is defined intrinsically for any substance or category of substances to be defined as agonists, antagonists, or partial agonists. As used herein, "intrinsic activity", or "efficacy," relates to some measure of biological effectiveness of the binding partner complex. With regard to receptor pharmacology, the context in which intrinsic activity or efficacy should be defined will depend on the context of the binding partner (e.g., receptor/ligand) complex and the consideration of an activity relevant to a particular biological outcome. For example, in some circumstances, intrinsic activity may vary depending on the particular second messenger system involved. See Hoyer, D. and Boddeke, H., Trends Pharmacol Sci. 14(7):270-5 (1993). Where such contextually specific evaluations are relevant, and how they might be relevant in the context of the present invention, will be apparent to one of ordinary skill in the art.

As used herein, neurotransmitters whose release is mediated by the compounds described herein include, but are not limited to, acetylcholine, dopamine, norepinephrine, serotonin, and glutamate, and the compounds described herein function as agonists or partial agonists at one or more of the Central Nervous System (CNS) nAChRs.

### I. Compounds

The hydroxybenzoic acids that can be used to prepare the hydroxybenzoate salts of the (E)-metanicotine-type compound ((4E)-N-methyl-5-(5-isopropoxy-3-pyridinyl)-4-penten-2-amine) are selected from the group consisting of salicylic acid, meta-hydroxybenzoic acid, para-hydroxybenzoic acid, vanillic acid, isovanillic acid, gentisic acid, gallic acid, 5-aminosalicylic acid, syringic acid, 4-methylsalicylic acid, 3-chloro-4-hydroxybenzoic acid, and 5-hydroxyisophthalic acid.

### II. Compound Preparation

The manner in which the (E)-metanicotine-type compounds described herein are synthetically produced can vary.

The (E)-metanicotine-type compounds possess a branched side chain, (4E)-N-methyl-5-(5-isopropoxy-3-pyridinyl)-4-penten-2-amine. By using one synthetic approach, the latter compound can be synthesized in a convergent manner, in which the side chain, N-methyl-N-(tert-butoxycarbonyl)-4-penten-2-amine is coupled with the 3-substituted 5-halo-substituted pyridine, 5-bromo-3-isopropoxypyridine, under Heck reaction conditions, followed by removal of the tert-butoxycarbonyl protecting group. Typically, the types of procedures set forth in W. C. Frank et al., J. Org. Chem. 43:2947 (1978) and N. J. Malek et al., J. Org. Chem. 47:5395 (1982) involving a palladium-catalyzed coupling of an olefin and an aromatic halide are used. The required N-methyl-N-(tert-butoxycarbonyl)-4-penten-2-amine can be synthesized as follows: (i) commercially available 4-penten-2-ol (Aldrich Chemical Company, Lancaster Synthesis Inc.) can be treated with p-toluenesulfonyl chloride in pyridine to yield 4-penten-2-ol p-toluenesulfonate, previously described by T. Michel, et al., Liebigs Ann. 11: 1811 (1996); (ii) the resulting tosylate can be heated with excess methylamine to yield N-methyl-4-penten-2-amine; (iii) the resulting amine, such as previously mentioned by A. Viola et al., J. Chem. Soc., Chem. Commun. 21: 1429 (1984), can be allowed to react with 1.2 molar equivalents of di-tert-butyl dicarbonate in dry tetrahydrofuran to yield the side chain, N-methyl-N-(tert-butoxycarbonyl)-4-penten-2-amine. The halo-substituted pyridine (e.g., 5-bromo-3-isopropoxypyridine), can be synthesized by at least two different routes. In one preparation, 3,5-dibromopyridine is heated at 140° C for 14 hours with 2 molar equivalents of potassium isopropoxide in dry isopropanol in the presence of copper powder (5%, w/w of the 3,5-dibromopyridine) in a sealed glass tube to yield 5-bromo-3-isopropoxypyridine. A second preparation of 5-bromo-3-isopropoxypyridine from 5-bromonicotinic acid can be performed as follows: (i) 5-Bromonicotinic acid is converted to 5-bromonicotinamide by treatment with thionyl chloride, followed by reaction of the intermediate acid chloride with aqueous ammonia. (ii) The resulting 5-bromonicotinamide, previously described by C. V. Greco et al., J. Heteocyclic Chem. 7(4):761 (1970), is subjected to Hofmann degradation by treatment with sodium hydroxide and a 70% solution of calcium hypochlorite. (iii) The resulting 3-amino-5-bromopyridine, previously described by C. V. Greco et al., J. Heteocyclic Chem. 7(4): 761 (1970), can be converted to 5-bromo-3-isopropoxypyridine by diazotization with isoamyl nitrite under acidic conditions, followed by treatment of the intermediate diazonium salt with isopropanol to yield 5-bromo-3-isopropoxypyridine. The palladium-catalyzed coupling of 5-bromo-3-isopropoxypyridine and N-methyl-N-(tert-butoxycarbonyl)-4-penten-2-amine is carried out in acetonitrile-triethylamine (2:1, v,v) using a catalyst consisting of 1 mole % palladium(II) acetate and 4 mole % tri-o-tolylphosphine. The reaction can be carried out by heating the components at 80° C for 20 hours to yield (4E)-N-methyl-N-(tert-butoxycarbonyl)-5-(5-isopropoxy-3-pyridinyl)-4-penten- 2-amine. Removal of the tert-butoxycarbonyl protecting group can be accomplished by treatment with 30 molar equivalents of trifluoroacetic acid in anisole at 0° C to afford (4E)-N-methyl-5-(5-isopropoxy-3-pyridinyl)-4-penten-2-amine. A variety of N-methyl-5-(5-alkoxy or 5-aryloxy-3-pyridinyl)-4-penten-2-amines are available from 3,5-dibromopyridine using this type of technology (i.e., treatment with sodium or potassium alkoxides or aryloxides and subsequent Heck coupling and deprotection).

In another synthetic approach, a compound such as (4E)-N-methyl-5-(5-methoxy-3-pyridinyl)-4-penten-2-amine (not covered by the invention) can be synthesized by coupling a halo-substituted pyridine, 5-bromo-3-methoxypyridine with an olefin containing a secondary alcohol functionality, 4-penten-2-ol, under Heck reaction conditions; and the resulting pyridinyl alcohol intermediate can be converted to its p-toluenesulfonate ester, followed by treatment with methylamine. Typically, the types of procedures set forth in W. C. Frank et al., J. Org. Chem. 43: 2947 (1978) and N. J. Malek et al., J. Org. Chem. 47: 5395 (1982) involving a palladium-catalyzed coupling of an olefin and an aromatic halide are used. The halo-substituted pyridine, 5-bromo-3-methoxypyridine is synthesized using methodology similar to that described by H. J. den Hertog et al., Recl. Trav. Chim. Pays-Bas 67:377 (1948), namely by heating 3,5-dibromopyridine with 2.5 molar equivalents of sodium methoxide in dry methanol in the presence of copper powder (5%, w/w of the 3,5-dibromopyridine) in a sealed glass tube at 150° C for 14 hours to produce 5-bromo-3-methoxypyridine. The resulting 5-bromo-3-methoxypyridine, previously described by D. L. Comins, et al., J. Org. Chem. 55: 69 (1990), can be coupled with 4-penten-2-ol in acetonitrile-triethylamine (1:1, v/v) using a catalyst consisting of I mole % palladium (II) acetate and 4 mole % tri-o-tolylphosphine. The reaction is carried out by heating the components in a sealed glass tube at 140° C for 14 hours to yield (4E)-5-(5-methoxy-3-pyridinyl)-4-penten-2-ol. The resulting alcohol is treated with 2 molar equivalents of p-toluenesulfonyl chloride in dry pyridine at 0°C to produce (4E)-5-(5-methoxy-3-pyridinyl)-4-penten-2-ol p-toluensulfonate. The tosylate intermediate is treated with 120-molar equivalents of methylamine as a 40% aqueous solution, containing a small amounts of ethanol as a co-solvent to product (4E)-N-methyl-5-(5-methoxy-3-pyridinyl)-4-penten-2-amine.

Optically active forms of certain aryl substituted olefinic amine compounds, such as (2S)-(4E)-N-methyl-5-(3-pyridinyl)-4-penten-2-amine, can be provided. In one synthetic approach, the latter type of compound is synthesized by coupling a halo-substituted pyridine, 3-bromopyridine, with an olefin possessing a chiral, secondary alcohol functionality, (2R)-4-penten-2-ol, under Heck reaction conditions. The resulting chiral pyridinyl alcohol intermediate, (2R)-(4E)-5-(3-pyridinyl)-4-penten-2-ol is converted to its corresponding p-toluenesulfonate ester, which is subsequently treated with methylamine, resulting in tosylate displacement with inversion of configuration. Typically, the types of procedures set forth in W. C. Frank et al., J. Org. Chem. 43: 2947 (1978) and N. J. Malek et al., J. Org Chem, 47: 5395 (1982) involving a palladium-catalyzed coupling of an aromatic halide and an olefin are used. The chiral side chain, (2R)-4-penten-2-ol can be prepared by treatment of the chiral epoxide, (R)-(+)-propylene oxide (commercially available from Fluka Chemical Company) with vinylmagnesium bromide and copper(I) iodide in tetrahydrofuran at low temperatures (-25 to -10° C) using the general synthetic methodology of A. Kalivretenos, J. K. Still, and L. S. Hegedus, J. Org. Chem. 56: 2883 (1991), to afford (2R)-4-penten-2-ol. The resulting chiral alcohol is subjected to a Heck reaction with 3-bromopyridine in acetonitrile-triethylamine (1:1, v/v) using a catalyst consisting of 1 mole % palladium(II) acetate and 4 mole % tri-o-tolylphosphine. The reaction is done by heating the components at 140° C for 14 hours in a sealed glass tube, to produce the Heck reaction product, (2R)-(4E)-5-(3-pyridinyl)-4-penten-2-ol. The resulting chiral pyridinyl alcohol is treated with 3 molar equivalents of p-toluenesulfonyl chloride in dry pyridine at 0° C, to afford the tosylate intermediate. The p-toluenesulfonate ester is heated with 82 molar equivalents of methylamine as a 40% aqueous solution, containing a small amount of ethanol as a co-solvent, to produce (2S)-(4E)-N-methyl-5-(3-pyridinyl)-4-penten-2-amine.

In a similar manner, the corresponding aryl substituted olefinic amine enantiomer, such as (2R)-(4E)-N-methyl-5-(3-pyridinyl)-4-penten-2-amine, can be synthesized by the Heck coupling of 3-bromopyridine and (2S)-4-penten-2-ol. The resulting intermediate, (2S)-(4E)-5-(3-pyridinyl)-4-penten-2-ol, is converted to its p-toluenesulfonate, which is subjected to methylamine displacement. The chiral alcohol, (2S)-4-penten-2-ol, is prepared from (S)-(-)-propylene oxide (commercially available from Aldrich Chemical Company) using a procedure analogous to that described for the preparation of (2R)-4-penten-2-ol from (R)-(+)-propylene oxide as reported by A. Kalivretenos, J. K. Stille, and L. S. Hegedus, J. Org. Chem. 56: 2883 (1991).

These methods each provide the (E)-metanicotine-type compounds as the major product, but also produce a minor amount of the corresponding (Z)-metanicotine-type compounds and other isomers, as before described. These minor reaction products can be removed using conventional techniques, if desired. Alternatively, as described in more detail below, the (E)-metanicotine-type compounds can be isolated as the hydroxybenzoate salts, which can precipitate out in substantially pure form from a reaction mixture including hydroxybenzoate salts of the (Z)-metanicotine-type compounds and other minor reaction products.

### Formation of E-metanicotine Hydroxybenzoates

The (E)-metanicotine hydroxybenzoates are formed by reacting the E-metanicotine-type compounds described above with hydroxybenzoic acids. The stoichiometry of the individual components (E-metanicotine and hydroxybenzoic acid) used to prepare the salts can vary. The molar ratio of hydroxybenzoic acid to base (E-metanicotine) is 2:1 to 1:2, more typically 2:1 or 1:1. It is preferred that the molar ratio of acid to base is 1:1. Depending upon the manner by which the salts of the present invention are formed, those salts may have crystal strictures that may occlude solvents that are present during salt formation. Thus, salts of the present invention can occur as hydrates and other solvates of varying stoichiometry of solvent relative to aryl substituted amine.

The method for providing compounds, of the present invention can vary. For instance, the preparation of (2S)-(4E)-N-methyl-5-(5-isopropoxy-3-pyridinyl)-4 penten-2-amine in a p-hydroxybenzoate form can involve (i) adding a solution of suitably pure compound dissolved in ethanol to a solution of p-hydroxybenzoic acid (11 equivalents) in ethanol, heated under reflux, to form a precipitate (ii) applying heat and/or water and ethanol (water not to exceed 10%) to dissolve the precipitate, (iii) cooling the resulting solution if necessary to cause precipitation of the salt and (iv) filtering and collecting the salt. The stoichiometry, solvent mix, solute concentration and temperature employed can vary, but the formation of the salts is within the levels of skill of those of skill in the art.

### Formation of Other Salt Forms

If desired, once the hydroxybenzoate salts are isolated, other salt forms can be formed, for example, by direct reaction with another pharmaceutically acceptable acid or by first isolating the free base (by reaction with strong base and extraction into an appropriate solvent) and then reaction with another pharmaceutically acceptable acid. Such procedures are known to those of skill in the art.

### III. Pharmaceutical Compositions

The pharmaceutical compositions of the present invention include the hydroxybenzoates described herein, in the pure state or in the form of a composition in which the compounds are combined with any other pharmaceutically compatible product, which can be inert or physiologically active. Such compositions can be administered, for example, orally, parenterally, rectally, or topically.

Examples of solid compositions for oral administration include, but are not limited to, tablets, pills, powders (gelatin capsules, cachets), and granules. In these compositions, the active compound is mixed with one or more inert diluents, such as starch, cellulose, sucrose, lactose, or silica; ideally, under a stream of an inert gas such as argon.

The compositions can also include substances other than diluents, for example, one or more lubricants such as magnesium stearate or talc, a colorant, a coating (coated tablets), or a varnish.

Examples of liquid compositions for oral administration include, but are not limited to, solutions, suspensions, emulsions, syrups, and elixirs that are pharmaceutically acceptable and typically contain inert diluents such as water, ethanol, glycerol, vegetable oils, or liquid paraffin. These compositions can comprise substances other than the diluents, for example, wetting agents, sweeteners, thickeners, flavors, and stabilizers.

Sterile compositions for parenteral administration can include, for example, aqueous or nonaqueous solutions, suspensions, and emulsions. Examples of suitable solvents and vehicles include, but are not limited to aqueous solutions, preferably buffered aqueous solutions, propylene glycol, a polyethylene glycol, vegetable oils, especially olive oil, injectable organic esters, for example ethyl oleate, and other appropriate organic solvents. These compositions can also include adjuvants, especially wetting agents, isotonicity agents, emulsifiers, dispersants, and stabilizers. Such sterile compositions can be sterilized in a number of ways, for example, by asepticizing filtration, by incorporating sterilizing agents into the composition, by irradiation and by heating. They can also be prepared in the form of sterile solid compositions which can be dissolved at the time of use in sterile water or any other sterile injectable medium.

Examples of compositions for rectal administration include, but are not limited to, suppositories and rectal capsules that, in addition to the active product, can include excipients such as cocoa butter, semi-synthetic glycerides, and polyethylene glycols.

Compositions for topical administration can, for example, be creams, lotions, eyewashes, collutoria, nasal drops or aerosols.

The pharmaceutical compositions also can include various other components as additives or adjuncts. Exemplary pharmaceutically acceptable components or adjuncts which are employed in relevant circumstances include antioxidants, free radical scavenging agents, peptides, growth factors, antibiotics, bacteriostatic agents, immunosuppressives, anticoagulants, buffering agents, anti-inflammatory agents, anti-pyretics, time release binders, anesthetics, steroids, and corticosteroids. Such components can provide additional therapeutic benefit, act to affect the therapeutic action of the pharmaceutical composition, or act towards preventing any potential side effects which may be posed as a result of administration of the pharmaceutical composition. In certain circumstances, a compound of the present invention can be employed as part of a pharmaceutical composition with other compounds intended to prevent or treat a particular disorder.

### IV. Methods of Treatment

The hydroxybenzoate salts described herein are useful for treating those types of conditions and disorders for which other types of nicotinic compounds have been proposed as therapeutics. See, for example, Williams et al., DN&P 7(4):205-227 (1994); Arneric et al., CNS Drug Rev. 1(1):1-26 (1995); Arneric et al., Exp. Opin. Invest. Drugs 5(1):79-100 (1996); Bencherif et al., J. Pharmacol. Exp. Ther. 279:1413 (1996); Lippiello et al., J. Pharmacol. Exp. Ther. 279:1422 (1996); Damaj et al., Neuroscience (1997); Holladay et al., J. Med. Chem. 40(28): 4169-4194 (1997); Bannon et al., Science 279: 77-80 (1998); PCT WO 94/08992; PCT WO 96/31475; and U.S. Patent Nos. 5,583,140 to Bencherif et al.*;* 5,597,919 to Dull et al.*;* and 5,604,231 to Smith et al.

The salts can also be used as adjunct therapy in combination with existing therapies in the management of the aforementioned types of diseases and disorders. In such situations, it is preferably to administer the active ingredients in a manner that minimizes effects upon nAChR subtypes such as those that are associated with muscle and ganglia. This can be accomplished by targeted drug delivery and/or by adjusting the dosage such that a desired effect is obtained without meeting the threshold dosage required to cause significant side effects. The pharmaceutical compositions can be used to ameliorate any of the symptoms associated with those conditions, diseases, and disorders.

Examples of conditions and disorders that can be treated include neurological disorders, neurodegenerative disorders, in particular, CNS disorders, and inflammatory disorders. CNS disorders can be drug induced; can be attributed to genetic predisposition, infection or trauma; or can be of unknown etiology. CNS disorders comprise neuropsychiatric disorders, neurological diseases, and mental illnesses, and include neurodegenerative diseases, behavioral disorders, cognitive disorders, and cognitive affective disorders. There are several CNS disorders whose clinical manifestations have been attributed to CNS dysfunction (*i.e.,* disorders resulting from inappropriate levels of neurotransmitter release, inappropriate properties of neurotransmitter receptors, and/or inappropriate interaction between neurotransmitters and neurotransmitter receptors). Several CNS disorders can be attributed to a deficiency of choline, dopamine, norepinephrine, and/or serotonin.

Examples of CNS disorders that can be treated using the E-metanicotine compounds and hydroxybenzoate salts described herein, and pharmaceutical compositions including these compounds and salts, include pre-senile dementia (early onset Alzheimer's disease), senile dementia (dementia of the Alzheimer's type), Lewy Body dementia, micro-infarct dementia, AIDS-related dementia, HIV-dementia, multiple cerebral infarcts, Parkinsonism including Parkinson's disease, Pick's disease, progressive supranuclear palsy, Huntington's chorea, tardive dyskinesia, hyperkinesia, epilepsy, mania, attention deficit disorder, anxiety, depression, dyslexia, schizophrenia depression, obsessive-compulsive disorders, Tourette's syndrome, mild cognitive impairment (MCI), age-associated memory impairment (AAMI), premature amnesic and cognitive disorders which are age-related or a consequence of alcoholism, or immunodeficiency syndrome, or are associated with vascular disorders, with genetic alterations (such as, for example, trisomy 21) or with attention deficiencies or learning deficiencies, acute or chronic neurodegenerative conditions such as amyotrophic lateral sclerosis, multiple sclerosis, peripheral neurotrophies, and cerebral or spinal traumas. In addition, the compounds can be used to treat nicotine addiction and/or other behavioral disorders related to substances that lead to dependency (e.g., alcohol, cocaine, heroin and opiates, psychostimulants, benzodiazepines, and barbiturates), and to treat obesity. The compounds can also be used to treat pathologies exhibiting an inflammatory character within the gastrointestinal system such as Crohn's disease, irritable bowel syndrome and ulcerative colitis, and diarrheas.

The manner in which the hydroxybenzoate salts are administered can vary. The salts can be administered by inhalation (*e.g.*, in the form of an aerosol either nasally or using delivery articles of the type set forth in U.S. Patent No. 4,922,901 to Brooks et al.); topically (*e.g*., in lotion form); orally (*e.g.*, in liquid form within a solvent such as an aqueous or nonaqueous liquid, or within a solid carrier); intravenously (*e.g.*, within a dextrose or saline solution); as an infusion or injection (*e.g*., as a suspension or as an emulsion in a pharmaceutically acceptable liquid or mixture of liquids); intrathecally; intracerebroventricularly; or transdermally (*e.g.*, using a transdermal patch). Although it is possible to administer the salts in the form of a bulk active chemical, it is preferred to present each salts in the form of a pharmaceutical composition or formulation for efficient and effective administration. Exemplary methods for administering such salts will be apparent to the skilled artisan. For example, the salts can be administered in the form of a tablet, a hard gelatin capsule or as a time-release capsule. As another example, the salts can be delivered transdermally using the types of patch technologies available from Novartis and Alza Corporation. The administration of the pharmaceutical compositions of the present invention can be intermittent, or at a gradual, continuous, constant or controlled rate to a warm-blooded animal, (e.g., a mammal such as a mouse, rat, cat, rabbit, dog, pig, cow, or monkey); but, advantageously, the compounds are preferably administered to a human being. In addition, the time of day and the number of times per day that the pharmaceutical formulation is administered can vary. Administration preferably is such that the active ingredients of the pharmaceutical formulation interact with receptor sites within the body of the subject that affect the functioning of the CNS or of the gastrointestinal (GI) tract. More specifically, in treating a CNS disorder administration preferably is such so as to optimize the effect upon those relevant receptor subtypes which have an effect upon the functioning of the CNS, while minimizing the effects upon muscle-type receptor subtypes. Other suitable methods for administering the salts are described in U.S. Patent No. 5,604,231 to Smith et al.

The appropriate dose of the salts is that amount effective to prevent occurrence of the symptoms of the disorder or to treat some symptoms of the disorder from which the patient suffers. By "effective amount," "therapeutic amount," or "effective dose" is meant that amount sufficient to elicit the desired pharmacological or therapeutic effects, thus resulting in effective prevention or treatment of the disorder. Thus, when treating a CNS disorder, an effective amount of the hydroxybenzoate salts is an amount required to deliver, across the blood-brain barrier of the subject, a sufficient amount of the free base drug to bind to relevant receptor sites in the brain of the subject, and to modulate relevant nicotinic receptor subtypes (*e.g*., provide neurotransmitter secretion, thus resulting in affective prevention or treatment of the disorder). Prevention of the disorder is manifested by at least delaying the onset of the symptoms of the disorder or reducing the severity of the symptoms. Treatment of the disorder is manifested by a decrease in the symptoms associated with the disorder or an amelioration of the recurrence of the symptoms of the disorder.

The affective dose can vary, depending upon factors such as the condition of the patient, the severity of the symptoms of the disorder, and the manner in which the pharmaceutical composition is administered. For human patients, the effective dose of typical salts generally requires administering the salts in an amount sufficient to modulate relevant receptors to affect neurotransmitter (*e.g.*, dopamine) release but the amount should be insufficient to induce effects on skeletal muscles and ganglia to any significant degree. The effective dose of he hydroxybenzoate salts will of course differ from patient to patient but in general includes amounts starting where CNS effects or other desired therapeutic effects occur, but below the amount where muscular effects are observed.

The doses depend on the desired effect, the duration of treatment and the administration route used; they are generally between 0.05 mg and 100 mg of active substance per day orally for an adult. Generally speaking, a medical doctor will determine the appropriate dosage as a function of the age, weight and all the other factors specific to the patient.

The salts of the present invention, when employed in effective amounts, often lack the ability to elicit activation of human ganglion nAChRs to any significant degree. This selectivity of the salts of the present invention against those nAChRs responsible for cardiovascular side effects is demonstrated by a lack of the ability of those salts to activate nicotinic function of adrenal chromaffin tissue. As such, such salts have poor ability to cause isotopic rubidium ion flux through nAChRs in cell preparations derived from the adrenal gland. Generally, typical preferred salts useful in carrying out the present invention maximally activate isotopic rubidium ion flux by less than 10 percent, often by less than 5 percent, of that maximally provided by S(-) nicotine.

The salts are effective towards providing some degree of prevention of the progression of CNS disorders, ameliorating the symptoms of CNS disorders, and ameliorating to some degree the recurrence of CNS disorders. However, such effective amounts of those salts are not sufficient to elicit any appreciable undesired nicotinic effects, as is demonstrated by decreased effects on preparations believed to reflect effects on the cardiovascular system, or effects to skeletal muscle. As such, administration of salts of the present invention provides a therapeutic window in which treatment of certain CNS disorders is provided, and undesired peripheral nicotinic effects/side effects are avoided. That is, an effective dose of a compound of the present invention is sufficient to provide the desired effects upon the CNS, but is insufficient (*i.e*., is not at a high enough level) to provide undesirable side effects. Preferably, effective administration of a compound of the present invention resulting in treatment of CNS disorders occurs upon administration of less than 1/3, frequently less than 1/5, and often less than 1/10, that amount sufficient to cause any side effects to a significant degree.

The following synthetic and analytical examples are provided to illustrate the present invention, and should not be construed as limiting thereof. In these examples, all parts and percentages are by weight, unless otherwise noted. Reaction yields are reported in mole percentages.

### Example 1: Synthesis of (2S)-(4E)- N-methyl-5-(5-isopropoxy-3-pyridinyl)-4-penten-2-amine p-hydroxybenzoate

### (2S)-(4E)-N-methyl-5-(5-isopropoxy-3-pyridinyl)-4-penten-2-amine p-hydroxybenzoate

p-Hydroxybenzoic acid (2.62 g, 19.0 mmol) was added in portions to a stirred solution (2S)-(4E)- N-methyl-5-(5-isopropoxy-3-pyridinyl)-4-penten-2-amine (4.79 g of 93% pure, 19.0 mmol) in isopropyl acetate (50 mL). During the addition, crystallization of salt was evident. After complete addition of the p-hydroxybenzoic acid, the suspension was heated near its boiling point as isopropanol was slowly added. After 15 mL of isopropanol had been added, complete dissolution was obtained. Cooling of the solution to ambient temperature (overnight) resulted in deposition of a crystalline mass, which was collected by suction filtration and air dried (4.03 g). A second crop (0.82 g) was isolated from the concentrated filtrate, by addition of acetone. The two crops of crystals were combined and recrystallized from acetone (50 mL). The solid was collected by suction filtration and dried in the vacuum oven (50°C) for 18 h. This left 4.24 g (60.0%) of white crystals (98+% pure by both GCMS and LCMS; m.p. 99-101°C).

### Example 2: Synthesis of (2S)-(4E)- N-methyl-5-(5-isopropoxy-3-pyridinyl)-4-penten-2-amine (via the Heck reaction with (S)-N-Methyl-N-(tert-butoxycarbonyl)-4-penten-2-amine) and the use of the p-hydroxybenzoate salt to facilitate isolation and purification of (2S)-(4E)- N-methyl-5-(5-isopropoxy-3-pyridinyl)-4-penten-2-amine:

### 3-Bromo-5-isopropoxypyridine

A 72 L reactor was charged successively with sodium tert-pentoxide (2.2 kg, 20 mol) and 1-methyl-2-pyrrolidinone (17.6 L). This mixture was stirred for 1 h, and then 2-propanol (12 L) was added over a period of 2 h. 3,5-Dibromopyridine (3.0 kg, 13 mol) was then added to the reactor, and the mixture was heated at 75°C for 12 h under a nitrogen atmosphere. The mixture was then cooled, diluted with toluene (15 L), and washed with water (30 L). The aqueous phase was extracted with toluene (15 L), and the combined toluene phases were washed with water (15 L) and concentrated under reduced pressure, to give 2.5 kg of dark oil. This was combined an equal sized batch of material from a second run and vacuum distilled (b.p. 65°C at 0.3 mm), to yield 3.1 kg (57%) of 3-bromo-5-isopropoxypyridine as a pale yellow oil.

### (2R)-4-Penten-2-ol

(2R)-4-Penten-2-ol was prepared in 82.5% yield from (R)-(+)-propylene oxide according to procedures set forth in A. Kalivretenos, J. K. Stille, and L. S. Hegedus, J. Org. Chem. 56: 2883 (1991).

### (S)-N-Methyl-N-(tert-butoxycarbonyl)-4-penten-2-amine

A mixture of (R)-4-penten-2-ol (7.62 g, 88.5 mmol), pyridine (15 mL), and dry (distilled from calcium hydride) dichloromethane (30mL) was stirred in an ice bath as p-toluenesulfonyl chloride (18.6 g, 97.4 mmol) was added over a 3 min period. The mixture was stirred 20 min at 0°C and 16 h at ambient temperature, as a heavy precipitate formed. Saturated aqueous sodium bicarbonate (75 mL) was added, and the biphasic mixture was stirred vigorously for 3 h. The dichloromethane phase and two dichloromethane extracts (50 mL each) of the aqueous phase were combined, dried (Na₂SO₄), and concentrated by rotary evaporation. High vacuum treatment left 18.7g of light yellow oil, which was combined with dimethylformamide (DMF) (35 mL) and 40% aqueous methylamine (35 mL). This solution was stirred at ambient temperature for 48 h and then poured into a mixture of saturated aqueous sodium chloride (300 mL) and 2.5 M sodium hydroxide (50 mL). This mixture was extracted with ether (5 x 250 mL), and the ether extracts were dried (Na₂SO₄) and concentrated by rotary evaporation (from an ice cooled bath) to a volume of about 250 mL. The remaining solution was combined with di-tert-butyl dicarbonate (16.9 g, 77.4 mmol) and THF (100 mL), and the mixture was stirred at ambient temperature for 16 h. The volatiles were evaporated by rotary evaporation, and the residue was vacuum distilled at 5 mm pressure (bp 79-86°C), to give 7.74 g (43.9 % yield) of clear, colorless liquid.

### (2S)-(4E)-N-Methyl-5-(5-isopropoxy-3-pyridinyl)-4-penten-2-amine p-hydroxybenzoate

A mixture of 3-bromo-5-isopropoxypyridine (21.0 g, 97.2 mmol), (S)-N-Methyl-N-(tert-butoxycarbonyl)-4-penten-2-amine (24.0 g, 120 mmol), DMF (53 mL), K₂CO₃ (22 g, 159 mmol), palladium(II) acetate (0.22 g, 0.98 mmol) and tri-o-tolylphosphine (0.57 g, 1.9 mmol) was degassed and placed under a nitrogen atmosphere. The stirred mixture was then heated at 130°C for 2.5 h. To remove palladium salts, Smopex™ (20 g) and ethyl acetate (100mL) were added. The stirred mixture was heated at 50°C for 5 h and at ambient temperature for 16 h and then filtered. The filtrate was concentrated under reduced pressure, and the residue (83 g) was dissolved in methanol (25 mL), cooled in a cold water bath (<5°C) and treated drop-wise with 6 M HCl (100 mL). This mixture was stirred 3 h at ambient temperature, and the methanol was removed by concentration under vacuum. The remaining aqueous mixture was washed with dichloromethane (100 mL), made basic by careful (with cooling) addition of 3 M NaOH, and extracted (2 x 200 mL) with dichloromethane. These latter extracts were washed with saturated aqueous NaCl and concentrated under vacuum. The residue was dissolved in acetone (150 mL), and p-hydroxybenzoic acid (14.0 g, 101 mmol) was added. After complete dissolution of the p-hydroxybenzoic acid, the solution was kept at ambient temperature, as a large amount of solid formed (several hours). After several hours of cooling at -15°C, the mixture was suction filtered. The resulting solid (24.8 g) was recrystallized from acetone (240 mL) to give 22.3 g (61.6%) of off-white crystals (97+% pure by GCMS and LCMS).

### Example 3: Synthesis of (2S)-(4E)-N-methyl-5-(5-methoxy-3-pyridinyl)-4-penten-2- amine 2,5-dihydroxybenzoate (gentisate) (not part of the invention)

### (2S)-(4E)-N-Methyl-5-(5-methoxy-3-pyridinyl)-4-penten-2-amine 2.5-dihvdroxybenzoate

A hot solution of 2,5-dihydroxybenzoic acid (gentisic acid) (0.582 g, 3.78 mmol) in absolute ethanol (1 mL) was added to a warm solution of (2S)-(4E)-N-methyl-5-(5-methoxy-3-pyridinyl)-4-penten-2-amine (1.00 g, 4.85 mmol, 86.7% E isomer by GC-FID) in absolute ethanol (1 mL), using additional ethanol (2 mL) in the transfer. The resulting mixture was concentrated via rotary evaporation, leaving 1.5 mL of ethanol in the solution. With stirring and heating to near reflux, crystallization occurred. The resulting hot mixture was treated drop-wise with ethyl acetate (5.5 mL). After cooling to room temperature, the mixture was further cooled at 5°C for 48 h. The resulting solids were filtered, washed with ethyl acetate (2 x 5 mL) and dried at 50°C to give 1.24 g (91%) of an off-white powder (98.0% E isomer by GC-FID for the free base). To remove the color from the sample, the material was recrystallized from ethanol/isopropanol (3.5 mL : 5.5 mL) to give 1.03 g (83% recovery) of an off-white powder and subsequently recrystallized from ethanol/ethyl acetate (3 mL : 12 mL) to give 0.90 g (87% recovery) of a white, crystalline powder, mp 166-167°C.

### Example 4: Synthesis of E-metanicotine 2,5-dihydroxybenzoate (not part of the invention) E-Metanicotine 2,5-dihydroxybenzoate

2,5-Dihydroxybenzoic acid (gentisic acid) (0.475 g, 3.08 mmol) was added to a solution of E-metanicotine (0.500 g, 3.08 mmol) in ethyl acetate (3 mL) and isopropanol (2.5 mL), and the resulting mixture was gently heated until all solids dissolved. Upon cooling, a white granular precipitate was deposited, and the mixture was copied at 5°C. The solids were filtered, washed with cold isopropanol (3 x 2 mL) and dried under vacuum at 40°C for 4 h to give 0.58 g (29.7%) of a light-yellow, flaky solid, mp 90-91.5°C. ¹H NMR (D₂O): mono-salt stoichiometry. Calcd. for C₁₀H₁₄N₂· C₇H₆O4· 0.15 H₂O: C, 64.00%; H, 6.41%; N, 8.78%. Found: C, 63.92, 64.00%; H, 6.33, 6.34%; N, 8.79, 8.84%.

### Example 5: Synthesis of E-metanicotine 3,5-dihydroxybenzoate (not part of the invention) E-Metanicotine 3,5-dihydroxybenzoate

3,5-Dihydroxybenzoic acid (0.475 g, 3.08 mmol) was added to a warm solution of E-metanicotine (0.500 g, 3.08 mmol) in isopropanol (11 mL) and methanol (4.5 mL). Upon heating to near reflux to dissolve the resulting gum, the light-yellow solution was cooled to room temperature and further cooled at 5°C. The resulting dark-yellow gum that was deposited was dissolved in isopropyl acetate (3 mL) and methanol (4 mL), assisted by heating. After cooling to room temperature and further cooling at 5°C, the off-white solids were filtered, washed with isopropyl acetate and dried to give 0.505 g (51.8 %) of waxy, tan flakes, mp 160-161.5°C. ¹H NMR (D₂O): mono-salt stoichiometry. Calcd. for C₁₀H₁₄N₂ · C₇H₆O₄ · 0.15H₂O: C, 64.00%; H, 6.41%; N, 8.78%. Found: C, 64.03, 64.02%; H, 6.38, 6.38%; N, 8.80, 8.76%.

### Analytical Examples

### Example 6: Determination of Binding to Relevant Receptor Sites

The interaction of the hydroxybenzoate salts with relevant receptor sites can be determined in accordance with the techniques described in U.S. Pat. No. 5,597,919 to Dull et al. Inhibition constants (Ki values), reported in nM, can be calculated from the IC₅₀ values using the method of Cheng et al., Biochem, Pharnacol. 22:3099 (1973). Low binding constants indicate that the components of the salts described herein exhibit good high affinity binding to certain CNS nicotinic receptors.

## Claims

1. A salt formed as the reaction product of (4E)-N-methyl-5-(5-isopropoxy-3-pyridinyl)-4-penten-2-amine and a hydroxybenzoic acid, where the hydroxybenzoic acid is selected from the group consisting of salicylic acid, meta-hydroxybenzoic acid, para-hydroxybenzoic acid, vanillic acid, isovanillic acid, gentisic acid, gallic acid, 5-aminosalicylic acid, syringic acid, 4-methylsalicylic acid, 3-chloro-4-hydroxybenzoic acid and 5-hydroxyisophthalic acid, and wherein the molar ratio of (4E)-N-methyl-5-(5-isopropoxy-3-pyridinyl)-4-penten-2-amine to hydroxybenzoic acid ranges from 1:2 to 2:1.

2. The salt of Claim 1, wherein the hydroxybenzoic acid is o-, m- or p-hydroxybenzoic acid.

3. The salt of Claim 1, wherein the hydroxybenzoic acid is gentisic acid (2,5-dihydroxybenzoic acid).

4. The salt of Claim 1, wherein the (4E)-N-methyl-5-(5-isopropoxy-3-pyridinyl)-4-penten-2-amine is (2S)-(4E)-N-methyl-5-(5-isopropoxy-3-pyridinyl)-4-penten-2-amine.

5. A pharmaceutical composition comprising a salt of any of Claims 1-4, along with a pharmaceutically acceptable carrier.

6. Use of a salt of any of Claims 1-4, and a pharmaceutically acceptable carrier, in the preparation of a medicament for treating a CNS disorder.

7. A process for preparing (2S)-(4E)-N-methyl-5-(5-isopropoxy-3-pyridinyl)-4-penten-2-amine or a corresponding hydroxybenzoate salt, comprising the steps of:
a) performing a Heck coupling reaction between a 3-halo-5-isopropoxypyridine and a compound of the formula (S)-CH₂=CH-CH₂-CH(CH₃)-N(CH₃)(pg), where pg is a protecting group for an amine, and
b) deprotecting the protected amine group, or
c) performing a Heck coupling reaction between a 3-halo-5-isopropoxypyridine and a compound of the formula (R)-CH₂=CH-CH₂-CH(CH₃)-OH and
d) converting the OH group to an NHCH₃ group,
wherein b) or d) result in a mixture of compounds including (2S)-(4E)-N-methyl-5-(5-isopropoxy-3-pyridinyl)-4-penten-2-amine, the related Z-metanicotine compound, and other isomers,
e) forming a hydroxybenzoate salt by reaction of the mixture with a hydroxybenzoic acid selected from the group consisting of salicylic acid, meta-hydroxybenzoic acid, para-hydroxybenzoic acid, vanillic acid, isovanillic acid, gentisic acid, gallic acid, 5-aminosalicylic acid, syringic acid, 4-methylsalicylic acid, 3-chloro-4-hydroxybenzoic acid and 5-hydroxyisophthalic,
wherein the molar ratio of (4E)-N-methyl-5-(5-isopropoxy-3-pyridinyl)-4-penten-2-amine to hydroxybenzoic acid ranges from 1:2 to 2:1,
f) isolating the (2S)-(4E)-N-methyl-5-(5-isopropoxy-3-pyridinyl)-4-penten-2-amine hydroxybenzoate salt, and
g) optionally converting the (2S)-(4E)-N-methyl-5-(5-isopropoxy-3-pyridinyl)-4-penten-2-amine hydroxybenzoate salt to (2S)-(4E)-N-methyl-5-(5-isopropoxy-3-pyridinyl)-4-penten-2-amine.

8. The method of Claim 7, wherein the hydroxybenzoic acid is o-, m- or p-hydroxybenzoic acid.

9. The method of Claims 7 or 8, wherein the hydroxybenzoate salt is converted to another pharmaceutically acceptable salt form.

10. The use of a composition comprising a salt of any of Claims 1-4 in the preparation of a medicament useful for treating a CNS disorder.

11. A composition comprising a salt of any of Claims 1-4, and optionally a pharmaceutically acceptable carrier, for use in treating a CNS disorder.

## Patentansprüche

1. Salz, das als das Reaktionsprodukt von (4E)-N-Methyl-5-(5-isopropoxy-3-pyridinyl)-4-penten-2-amin und einer Hydroxybenzoesäure gebildet wird, wobei die Hydroxybenzoesäure aus der Gruppe ausgewählt ist, die aus Salicylsäure, meta-Hydroxybenzoesäure, para-Hydroxybenzoesäure, Vanillinsäure, Isovanillinsäure, Gentisinsäure, Gallussäure, 5-Aminosalicylsäure, Syringasäure, 4-Methylsalicylsäure, 3-Chlor-4-hydroxybenzoesäure und 5-Hydroxyisophthalsäure besteht, und wobei das molare Verhältnis von (4E)-N-Methyl-5-(5-isopropoxy-3-pyridinyl)-4-penten-2-amin zu Hydroxybenzoesäure im Bereich von 1:2 bis 2:1 liegt.

2. Salz nach Anspruch 1, wobei die Hydroxybenzoesäure o-, m- oder p-Hydroxybenzoesäure ist.

3. Salz nach Anspruch 1, wobei die Hydroxybenzoesäure Gentisinsäure (2,5-dihydroxybenzoesäure) ist.

4. Salz nach Anspruch 1, wobei das (4E)-N-Methyl-5-(5-isopropoxy-3-pyridinyl)-4-penten-2-amin (2S)-(4E)-N-Methyl-5-(5-isopropoxy-3-pyridinyl)-4-penten-2-amin ist.

5. Pharmazeutische Zusammensetzung, umfassend ein Salz nach einem der Ansprüche 1-4 zusammen mit einem pharmazeutisch verträglichen Trägerstoff.

6. Verwenden eines Salzes nach einem der Ansprüche 1-4 und eines pharmazeutisch verträglichen Trägerstoffs bei der Herstellung eines Medikaments zur Behandlung einer ZNS-Störung.

7. Verfahren zur Herstellung von (2S)-(4E)-N-Methyl-5-(5-isopropxy-3-pyridinyl)-4-penten-2-amin oder einem entsprechenden Hydroxybenzoatsalz, umfassend die Schritte:
a) Durchführen einer Heck-Kopplungsreaktion zwischen einem 3-Halo-5-isopropoxypyridin und einer Verbindung der Formel (S)-CH₂=CH-CH₂-CH(CH₃)-N(CH₃)(pg), wobei pg eine Schutzgruppe für ein Amin ist, und
b) Entschützen der geschützten Amingruppe, oder
c) Durchführen einer Heck-Kopplungsreaktion zwischen einem 3-Halo-5-isopropoxypyridin und einer Verbindung der Formel (R)-CH₂=CH-CH₂-CH(CH₃)-OH, und
d) Umwandeln der OH-Gruppe in eine NHCH₃-Gruppe,
wobei b) oder d) zu einer Mischung aus Verbindungen führt, die (2S)-(4E)-N-Methyl-5-(5-isopropoxy-3-pyridinyl)-4-penten-2-amin, die verwandte Z-Metanicotinverbindung und andere Isomere mit einschließen,
e) Bilden eines Hydroxybenzoatsalzes durch Reaktion der Mischung mit einer Hydroxybenzoesäure, ausgewählt aus der Gruppe, die aus Salicylsäure, meta-Hydroxybenzoesäure, para-Hydroxybenzoesäure, Vanillinsäure, Isovanillinsäure, Gentisinsäure, Gallussäure, 5-Aminosalicylsäure, Syringasäure, 4-Methylsalicylsäure, 3-Chlor-4-hydroxybenzoesäure und 5-Hydroxyisophthalsäure besteht,
wobei das molare Verhältnis von (4E)-N-Methyl-5-(5-isopropoxy-3-pyridinyl)-4-penten-2-amin zu Hydroxybenzoesäure im Bereich von 1:2 bis 2:1 liegt,
f) Isolieren des (2S)-(4E)-N-Methyl-5-(5-isopropoxy-3-pyridinyl)-4-penten-2-aminhydroxybenzoatsalzes, und
g) wahlweises Umwandeln des (2S)-(4E)-N-Methyl-5-(5-isopropoxy-3-pyridinyl)-4-penten-2-amin-hydroxybenzoatsalzes in (2S)-(4E)-N-Methyl-5-(5-isopropoxy-3-pyridinyl)-4-penten-2-amin.

8. Verfahren nach Anspruch 7, wobei die Hydroxybenzoesäure o-, m- oder p-Hydroxybenzoesäure ist.

9. Verfahren nach Anspruch 7 oder 8, wobei das Hydroxybenzoatsalz in eine andere pharmazeutisch verträgliche Salzform umgewandelt wird.

10. Verwenden einer Zusammensetzung, umfassend ein Salz nach einem der Ansprüche 1-4, bei der Herstellung eines Medikaments, das hilfreich bei der Behandlung einer ZNS-Störung ist.

11. Zusammensetzung, umfassend ein Salz nach einem der Ansprüche 1-4 und wahlweise einen pharmazeutisch verträglichen Trägerstoff, zur Verwendung bei der Behandlung einer ZNS-Störung.

## Revendications

1. Sel formé sous forme de produit réactionnel de la (4E)-N-méthyl-5-(5-isopropoxy-3-pyridinyl)-4-pentèn-2-amine et d'un acide hydroxybenzoïque, où l'acide hydroxybenzoïque est choisi dans le groupe constitué par l'acide salicylique, l'acide méta-hydroxybenzoïque, l'acide para-hydroxybenzoïque, l'acide vanillique, l'acide isovanillique, l'acide gentisique, l'acide gallique, l'acide-5-aminosalicylique, l'acide syringique, l'acide 4-méthylsalicylique, l'acide 3-chloro-4-hydroxybenzoïque et l'acide 5-hydroxyisophtalique, et où le rapport molaire de la (4E)-N-méthyl-5-(5-isopropoxy-3-pyridinyl)-4-pentèn-2-amine à l'acide hydroxybenzoïque se situe entre 1:2 et 2:1.

2. Sel selon la revendication 1, dans lequel l'acide hydroxybenzoïque est l'acide o-, mou p-hydroxybenzoïque.

3. Sel selon la revendication 1, dans lequel l'acide hydroxybenzoïque est l'acide gentisique (acide 2,5-dihydroxybenzoïque).

4. Sel selon la revendication 1, dans lequel la (4E)-N-méthyl-5-(5-isopropoxy-3-pyridinyl)-4-pentèn-2-amine est la (2S)-(4E)-N-méthyl-5-(5-isopropoxy-3-pyridinyl)-4-pentèn-2-amine.

5. Composition pharmaceutique comprenant un sel selon l'une quelconque des revendications 1 à 4, conjointement avec un vecteur pharmaceutiquement acceptable.

6. Utilisation d'un sel selon l'une quelconque des revendications 1 à 4 et d'un vecteur pharmaceutiquement acceptable, dans la préparation d'un médicament pour traiter un trouble du SNC.

7. Procédé de préparation de (2S)-(4E)-N-méthyl-5-(5-isopropoxy-3-pyridinyl)-4-pentèn-2-amine ou d'un sel d'hydroxybenzoate correspondant, comprenant les étapes consistant à :
a) effectuer une réaction de couplage de Heck entre une 3-halo-5-isopropoxypyridine et un composé de formule (S)-CH₂=CH-CH₂-CH(CH₃)-N(CH₃)(pg), où pg est un groupe protecteur pour une amine, et
b) déprotéger le groupe amine protégé, ou
c) effectuer une réaction de couplage de Heck entre une 3-halo-5-isopropoxypyridine et un composé de formule (R)-CH₂=CH-CH₂-CH(CH₃)-OH et
d) convertir le groupe OH en un groupe NHCH₃,
où b) ou d) produit un mélange de composés comprenant de la (2S)-(4E)-N-méthyl-5-(5-isopropoxy-3-pyridinyl)-4-pentèn-2-amine, le composé Z-métanicotine apparenté et d'autres isomères,
e) former un sel d'hydroxybenzoate par réaction du mélange avec un acide hydroxybenzoïque choisi dans le groupe constitué par l'acide salicylique, l'acide méta-hydroxybenzoïque, l'acide para-hydroxybenzoïque, l'acide vanillique, l'acide isovanillique, l'acide gentisique, l'acide gallique, l'acide 5-aminosalicylique, l'acide syringique, l'acide 4-méthylsalicylique, l'acide 3-chloro-4-hydroxybenzoïque et l'acide 5-hydroxyisophtalique,
où le rapport molaire de la (4E)-N-méthyl-5-(5-isopropoxy-3-pyridinyl)-4-pentèn-2-amine à l'acide hydroxybenzoïque se situe entre 1:2 et 2:1,
f) isoler le sel d'hydroxybenzoate de (2S)-(4E)-N-méthyl-5-(5-isopropoxy-3-pyridinyl)-4-pentèn-2-amine, et
g) facultativement convertir le sel d'hydroxybenzoate de (2S)-(4E)-N-méthyl-5-(5-isopropoxy-3-pyridinyl)-4-pentèn-2-amine en (2S)-(4E)-N-méthyl-5-(5-isopropoxy-3-pyridinyl)-4-pentèn-2-amine.

8. Procédé selon la revendication 7, dans lequel l'acide hydroxybenzoïque est l'acide o-, m- ou p-hydroxybenzoïque.

9. Procédé selon la revendication 7 ou 8, dans lequel le sel d'hydroxybenzoate est converti en une autre forme de sel pharmaceutiquement acceptable.

10. Utilisation d'une composition comprenant un sel selon l'une quelconque des revendications 1 à 4 dans la préparation d'un médicament utile pour traiter un trouble du SNC.

11. Composition comprenant un sel selon l'une quelconque des revendications 1 à 4, et facultativement un vecteur pharmaceutiquement acceptable, pour une utilisation dans le traitement d'un trouble du SNC.
